(19) Europäisches Patentamt
European Patent Office
Office européen des brevets

(11) Veröffentlichungsnummer: **0 183 993**
**A2**

# (12) EUROPÄISCHE PATENTANMELDUNG

(21) Anmeldenummer: **85113863.6**

(22) Anmeldetag: **31.10.85**

(51) Int. Cl.[4]: **A01N 43/90**, A01N 47/28, A01N 47/12, A01N 57/10, C07D 471/14, C07D 471/20, C07F 9/65, //(C07D471/14,235:00,221:00,209:00),(C07D471/20,235:00,221:00,209:00)

(30) Priorität: **16.11.84 CH 5490/84**
**27.08.85 CH 3675/85**

(43) Veröffentlichungstag der Anmeldung: **11.06.86 Patentblatt 86/24**

(84) Benannte Vertragsstaaten: **AT BE CH DE FR GB IT LI NL SE**

(71) Anmelder: **F. HOFFMANN-LA ROCHE & CO. Aktiengesellschaft**

**CH-4002 Basel(CH)**

(72) Erfinder: **Obrecht, Jean-Pierre, Dr.**
**Lilienweg 2**
**CH-8952 Schlieren(CH)**

(74) Vertreter: **Urech, Peter, Dr. et al**
**Grenzacherstrasse 124 Postfach 3255**
**CH-4002 Basel(CH)**

## (54) 2H-Imidazo[1',2':1,2]pyrrolo[3,4-b]pyridine und deren Verwendung als Unkrautbekämpfungsmittel.

(57) Die Erfindung betrifft neue Unkrautbekämpfungsmittel, die durch einen Gehalt an herbizid wirksamen Verbindungen der Formel

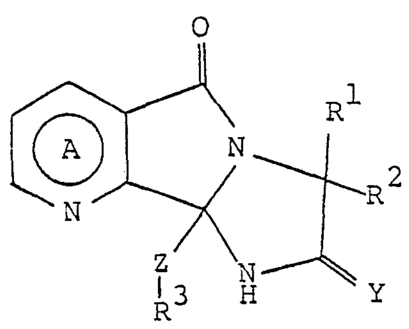

I

worin der Ring A, R¹, R², R³, Y und Z die in der Beschreibung angegebenen Bedeutungen besitzen, gekennzeichnet sind, und die Verwendung dieser Verbindungen und Mittel zur Unkrautbekämpfung. Die Erfindung betrifft ebenfalls neue Verbindungen der Formel I sowie deren Herstellung.

EP 0 183 993 A2

2H-Imidazo[1',2':1,2]pyrrolo[3,4-b]pyridine und deren Verwendung als Unkrautbekämpfungsmittel

Die vorliegende Erfindung betrifft Unkrautbekämpfungsmittel, die durch einen Gehalt an herbizid wirksamen heterocyclischen Verbindungen gekennzeichnet sind. Diese Verbindungen sind 2H-Imidazo[1',2':1,2]pyrrolo[3,4-b]pyridine der allgemeinen Formel

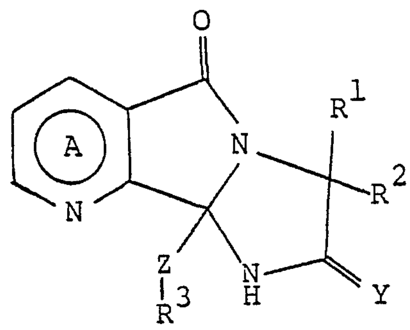

I

worin der

Pyridinring A gegebenenfalls substituiert sein kann, und

$R^1$ gegebenenfalls mit Fluor und/oder Chlor mono- oder mehrfach substituiertes $C_{1-4}$-Alkyl,

$R^2$ $C_{1-10}$-Alkyl oder $C_{3-6}$-Cycloalkyl, oder

$R^1$ und $R^2$ zusammen mit dem Kohlenstoffatom, an das sie geknüpft sind, einen gegebenenfalls mit einem oder zwei $C_{1-4}$-Alkylresten substituierten $C_{3-6}$-Cycloalkanring,

$R^3$ Wasserstoff; unsubstituiertes verzweigtes Alkyl mit bis zu 12 Kohlenstoffatomen; geradkettiges oder verzweigtes $C_{1-12}$-Alkyl, substituiert mit einem oder mehreren Halogenatomen, einer oder mehreren Hydroxylgruppen, einer Cyanogruppe, einer $C_{3-6}$-Cycloalkylgruppe, einer $C_{1-4}$-Alkoxygruppe, einer Pyridylgruppe, einer gegebenenfalls substituierten Phenoxygruppe, einer $\alpha$- oder $\beta$ -Naphthyloxygruppe oder einer der Gruppen (c) - (j)

$-CO-R^6$ (c)

worin

$R^6$ Wasserstoff, Hydroxy, $C_{1-4}$-Alkyl, $C_{1-4}$-Alkoxy oder Phenyl bedeutet,

$-SO_{n'}R^7$ (d)

worin

$R^7$ Hydroxy, Methyl, Phenyl oder p-Tolyl und

n' 0, 1 oder 2 bedeuten,

wobei, falls $R^7$ Hydroxy bedeutet, n' 2 bedeutet,

$-OSO_2R^7$ (e)

worin $R^7$ die oben angegebene Bedeutung besitzt,

einer gegebenenfalls veresterten Phosphit-, Phosphat- oder (f)

Phosphonatgruppe

insbesondere einer der Formel

$-(O)_{n''}P(O)_{n''}(R^8)_2$ (f')

worin die beiden

$R^8$ unabhängig voneinander Hydroxy, $C_{1-4}$ -Alkoxy oder Phenoxy

und die beiden

n" unabhängig voneinander 0 oder 1 bedeuten, wobei deren Summe 1 oder 2 beträgt,

$-NHCONHR^9$ (g)

worin $R^9$ Wasserstoff, $C_{1-4}$-Alkyl oder Phenyl bedeutet,

$-NHCOOR^{10}$ (h)

worin $R^{10}$ $C_{1-4}$ -Alkyl bedeutet,

$-OCONHR^9$ (i)

worin $R^9$ die oben angegebene Bedeutung besitzt,

$-OCO(CH_2)_{n'}COR^{11}$ (j)

worin

$R^{11}$ Hydroxy, $C_{1-4}$-Alkoxy, Phenoxy oder Benzyloxy bedeutet und

n' die oben angegebene Bedeutung besitzt,

$C_{3-10}$-Alkinyl; $C_{3-6}$-Cycloalkyl; oder eine Gruppe (a) oder (b)

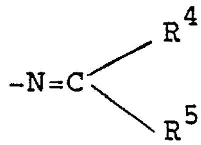

(a)

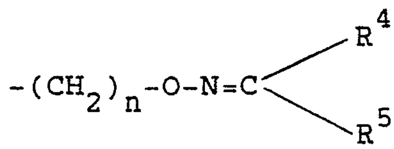

(b)

$R^4$ und $R^5$ unabhängig voneinander $C_{1-4}$-Alkyl,

n 1 oder 2 und

Y und Z unabhängig voneinander Sauerstoff oder Schwefel bedeuten,

mit Ausnahme von 1,9b-Dihydro-9b-hydroxy-3-isopropyl-3-methyl-2-thioxo-2H-imidazo[1',2':1,2]pyrrolo[3,4-b]pyridin-5(3H)-on, d.h. der Verbindung der Formel

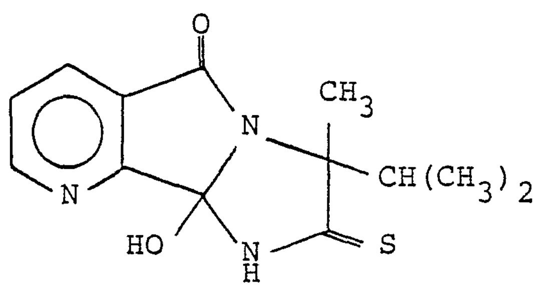

Der in der Formel I vorhandene Pyridinring A kann gegebenenfalls bis zu 3 Substituenten tragen. Es kommen als Substituenten insbesondere Halogen, $C_{1-6}$-Alkyl, Trifluormethyl, $C_{1-4}$-Hydroxyalkyl, $C_{1-6}$-Alkoxy, $C_{1-6}$-Alkylthio, Nitro, Cyano, Methylsulfonyl, Phenylsulfonyl, p-Tolylsulfonyl und gegebenenfalls substituiertes Phenyl, Phenoxy, Phenylthio und Benzyloxy in Frage. Die Substituenten können gleich oder verschiedenen sein. Ferner kann der Pyridinring A auch einen ankondensierten Benzol-, Pyridin- oder Pyrazinring als Substituent aufweisen, und zwar gemäss einer der nachfolgenden Teilstrukturen:

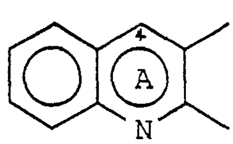

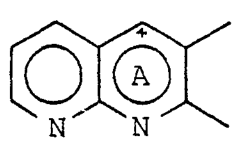

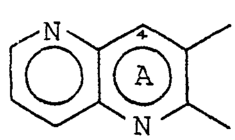

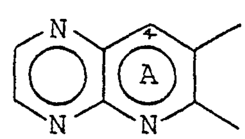

wobei der Ring A auch noch einen Substituenten in der 4-Stellung tragen kann, und zwar insbesondere einen der im Zusammenhang mit dem Pyridinring A oben genannten.

In den obigen Ausführungen betreffend die Substituenten des Pyridinrings A umfasst der Ausdruck "Halogen" Fluor, Chlor, Brom und Jod. Der Alkylrest kann geradkettig oder verzweigt sein, wobei dies auch für den Alkylteil der Hydroxyalkyl-, Alkoxy- bzw. Alkylthiogruppe gilt. Als Substituenten der substituierten Phenyl-, Phenoxy-, Phenylthio- bzw. Benzyloxygruppe kommen insbesondere 1 bis 3 Substituenten in Betracht, die vorzugsweise aus 1 bis 3 Halogenatomen, 1 oder 2 $C_{1-4}$-Alkylresten, einer Trifluormethylgruppe, 1 oder 2 $C_{1-4}$-Alkoxygruppen, 1 oder 2 Nitrogruppen und einer Cyanogruppe ausgewählt sind, wobei auch in diesem Fall "Halogen" Fluor, Chlor, Brom und Jod und die "Alkylreste" und "Alkoxygruppen" geradkettige und verzweigte Gruppen umfassen.

Die durch $R^1$ dargestellte Alkyl- oder Halogenalkylgruppe bzw. durch $R^2$, $R^4$ bzw. $R^5$ dargestellte Alkylgruppe kann geradkettig oder verzweigt sein, was auch für die Alkylsubstituenten des mit einem oder zwei $C_{1-4}$-Alkylresten substituierten $C_{3-6}$-Cycloalkanrings, den $R^1$ und $R^2$ zusammen mit dem diese tragenden Kohlenstoffatom bilden können, gilt.

In den obigen und folgenden Ausführungen betreffend die Substituenten des Alkylrestes $R^3$ ist unter "Halogen" jeweils Fluor, Chlor, Brom oder Jod zu verstehen. Eine allfällig vorhandene Alkyl- oder Alkoxygruppe kann geradkettig oder verzweigt sein.

Bedeutet $R^3$ Halogenalkyl oder Hydroxyalkyl, weist diese Gruppe vorzugsweise 1-5 Halogenatome bzw. 1-11, insbesondere 1-6 Hydroxygruppen, auf. Die Halogenatome sind vorzugsweise Fluor- und/oder Chloratome. Beispiele solcher Gruppen sind 2,2,2-Trifluoräthyl, 2-Chloräthyl und 2,2,3,3,3-Pentafluorpropyl bzw. 2-Hydroxyäthyl und 2,3-Dihydroxypropyl. Bedeutet $R^3$ Cycloalkylalkyl, so enthält dies vorzugsweise insgesamt 4-12 Kohlenstoffatome. Als Substituenten der substituierten Phenoxygruppe kommen insbesondere 1 bis 3 Substituenten in Betracht, die vorzugsweise aus 1-3 Halogenatomen, 1 oder 2 $C_{1-4}$-Alkylgruppen, einer Trifluormethylgruppe, 1 oder 2 $C_{1-4}$-Alkoxygruppen, 1 oder 2 Nitrogruppen und einer Cyanogruppe ausgewählt sind, wobei eine allfällig vorhandene Alkyl- oder Alkoxygruppe vorzugsweise Methyl bzw. Methoxy ist. Die Pyridylgruppe kann 2-, 3- oder 4-Pyridyl sein, jedoch ist sie vorzugsweise 3-Pyridyl.

Die oben definierte Carboxylgruppe (Gruppe (c), worin $R^6$ Hydroxy bedeutet); Sulfonsäuregruppe (Gruppe (d), worin $R^7$ Hydroxy bedeutet); Sulfatgruppe (Gruppe (e), worin $R^7$ Hydroxy bedeutet); gegebenenfalls veresterte Phosphit-, Phosphat- oder Phosphonatgruppe (f), worin mindestens eine Hydroxylgruppe vorhanden ist, insbesondere eine Gruppe (f'), worin mindestens eines der Symbole $R^8$ für Hydroxy steht; oder Carboxy(alkyl) carbonyloxygruppe (Gruppe (j), worin $R^{11}$ Hydroxy bedeutet) soll immer auch deren Metall- oder gegebenenfalls substituierte Ammoniumsalze umfassen, insbesondere die Alkalimetall-, wie Natrium- oder Kalium-, Erdalkalimetall-, wie Calcium- oder Magnesium-, Mangan-, Kupfer-, Eisen-, Zink-, Kobalt-, Blei-, Silber-, Nickel-, Ammonium- oder mono- oder mehrfach alkylierten Ammoniumsalze.

In der Gruppe (c) ist $R^6$ vorzugsweise Methyl oder Aethoxy und in der Gruppe (d) $R^7$ vorzugsweise Methyl und unabhängig davon n' vorzugsweise 0 oder 2. Falls die Gruppe (c), (d), (e), (f), (f') oder (j) in Form eines mono- oder mehrfach alkylierten Ammoniumsalzes vorliegt, so sind die Alkylsubstituenten insbesondere $C_{1-4}$-Alkylreste. Das substituierte Ammoniumion ist vorzugsweise Triäthylammonium. In der Gruppe (f') sind die beiden $R^8$ unabhängig voneinander vorzugsweise Wasserstoff, Methyl oder Aethyl, wobei ganz speziell bevorzugt die beiden $R^8$ die gleiche Bedeutung haben. Besonders bevorzugte Gruppen (f') sind die Phosphatgruppe und deren Dimethyl- und Diäthylester. Schliesslich bedeutet $R^9$, $R^{10}$, $R^9$ oder $R^{11}$ in der Gruppe (g), (h), (i) bzw. (j) vorzugsweise Methyl; Methyl; Wasserstoff, Methyl oder Phenyl; resp. Hydroxy oder ein Salz davon, insbesondere das Triäthylammoniumsalz.

Die durch $R^3$ dargestellte Alkinylgruppe kann geradkettig oder verzweigt sein und eine oder mehrere Dreifachbindungen aufweisen.

Ist der Pyridinring A substituiert, so sind die Substituenten vorzugsweise 1-3 Halogenatome, insbesondere Fluor, Chlor und/oder Brom, speziell bevorzugt ein Chloratom; 1 oder 2 Alkylreste, insbesondere ein Alkylrest, speziell bevorzugt Methyl oder Aethyl; eine Trifluormethylgruppe; eine Hydroxyalkylgruppe, insbesondere Hydroxymethyl; 1 oder 2 Alkoxygruppen, insbesondere eine Alkoxygruppe, speziell bevorzugt Aethoxy; eine Alkylthiogruppe, insbesondere Methylthio; 1 oder 2 Nitrogruppen, insbesondere eine Nitrogruppe; eine Cyanogruppe; und/oder eine gegebenenfalls substituierte Phenyl-, Phenoxy-, Phenylthio- oder Benzyloxygruppe. Vorzugsweise sind nicht mehr als zwei Substituenten vorhanden, speziell bevorzugt ein einziger Substituent, insbesondere Halogen, $C_{1-6}$-Alkyl oder $C_{1-6}$ -Alkoxy ist. Weist der Pyridinring A einen ankondensierten Benzol-, Pyridin- oder Pyrazinring auf, so ist dieser vorzugsweise ein Benzolring. Der Pyridinring A ist jedoch vorzugsweise unsubstituiert.

Unabhängig voneinander bedeuten $R^1$ vorzugsweise unsubstituiertes $C_{1-4}$-Alkyl, insbesondere Methyl; $R^2$ vorzugsweise $C_{1-10}$-Alkyl, insbesondere Isopropyl; und $R^3$ vorzugsweise Wasserstoff, gegebenenfalls substituiertes Alkyl, wie oben näher definiert, $C_{3-10}$-Alkinyl oder $C_{3-6}$-Cycloalkyl. Von den substituierten Alkylgruppen $R^3$ sind Hydroxyalkyl, Cyanoalkyl, Cycloalkylalkyl, Alkoxyalkyl, gegebenenfalls substituiertes Phenoxyalkyl und mit einer Gruppe (d) oder (h) substituiertes Alkyl bevorzugt, Hydroxyalkyl, Cycloalkylalkyl, Alkoxyalkyl und gegebenenfalls substituiertes Phenoxyalkyl besonders bevorzugt. Im Vordergrund des Interesses sind aber unsubstituiertes verzweigtes Alkyl, insbesondere Isopropyl und Isobutyl, sowie Alkoxyalkyl, insbesondere 2-Methoxy- und 2-Aethoxyäthyl.

Das Vorhandensein mindestens eines asymmetrischen Kohlenstoffatoms in den Verbindungen der Formel I hat zur Folge, dass die Verbindungen in optisch isomeren Formen auftreten können. Durch das Vorliegen einer allfälligen aliphatischen C=C-Doppelbindung kann auch geometrische Isomerie auftreten. Die Formel I soll all diese möglichen isomeren Formen umfassen.

Besonders bevorzugte Verbindungen der Formel I sind:

1,9b-Dihydro-9b-isopropoxy-3-isopropyl-3-methyl-2H-imidazo[1',2':1,2]pyrrolo[3,4-b]pyridin-2,5(3H)-dion,

1,9b-Dihydro-9b-isobutoxy-3-isopropyl-3-methyl-2H-imidazo-[1',2':1,2]pyrrolo[3,4-b]pyridin-2,5(3H)-dion,

1,9b-Dihydro-3-isopropyl-9b-(2-methoxyäthoxy)-3-methyl-2-H-imidazo[1',2':1,2]pyrrolo[3,4-b]pyridin-2,5(3H)-dion und

9b-(2-Aethoxyäthoxy)-1,9b-dihydro-3-isopropyl-3-methyl-2H--imidazo[1',2':1,2]pyrrolo[3,4-b]pyridin-2,5(3H)-dion.

Weitere Vertreter von Verbindungen der Formel I sind:

1,9b-Dihydro-9b-isopropoxy-3-isopropyl-3-methyl-2-thioxo-2-H-imidazo[1',2':1,2]pyrrolo[3,4-b]pyridin-5(3H)-on,

1,9b-Dihydro-3-isopropyl-3-methyl-9b-[2-(3-methylureido)-äthoxy]-2H-imidazo[1',2':1,2]pyrrolo[3,4-b]pyridin-2,5(3H)-dion,

1,9b-Dihydro-3-isopropyl-9b-[2-(isopropylidenamino)oxyäthoxy]-3-methyl-2H-imidazo[1',2':1,2]pyrrolo[3,4-b]pyridin-2,5-(3H)-dion,

1,9b-Dihydro-3-isopropyl-9b-[(isopropylidenamino)oxy]-3-methyl-2H-imidazo[1',2':1,2]pyrrolo[3,4-b]pyridin-2,5(3H)-dion,

1,9b-Dihydro-3-isopropyl-3-methyl-9b-{2-[(methylcarbamoyl)oxy]-äthoxy}-2H-imidazo[1',2':1,2]pyrrolo[3,4-b]pyridin-2,5-(3H)-dion,

1,9b-Dihydro-3-isopropyl-3-methyl-9b-[2-(sulfooxy)-äthoxy]--2H-imidazo[1',2':1,2]pyrrolo[3,4-b]pyridin-2,5(3H)-dion-Natriumsalz,

1,9b-Dihydro-3-isopropyl-3-methyl-9b-[2-(phosphonooxy)-äthoxy]-2H-imidazo[1',2':1,2]pyrrolo[3,4-b]pyridin-2,5(3H)-dio-

n,

1,9b-Dihydro-3-isopropyl-3-methyl-9b-(3,3,3-trifluorpropoxy)-2H-imidazo[1',2':1,2]pyrrolo[3,4-b]pyridin-2,5(3H)-dion,

1,9b-Dihydro-9b-(2,3-dihydroxypropoxy)-3-isopropyl-3-methyl-2H-imidazo[1',2':1,2]pyrrolo[3,4-b]pyridin-2,5(3H)-dion,

9b-{2-[[2-[(Benzyloxy)carbonyl]äthyl]carbonyloxy]-äthoxy}--1,9b-dihydro-3-isopropyl-3-methyl-2H-imidazo-[1',2':1,2]pyrrolo[3,4-b]pyridin-2,5(3H)-dion,

9b-{2-[[(2-Carboxyäthyl)carbonyl]oxy]-äthoxy}-1,9b-dihydro-3-isopropyl-3-methyl-2H-imidazo[1',2':1,2]pyrrolo[3,4-b]pyridin-2,5(3H)-dion,

9b-{2-[[(2-Carboxyäthyl)carbonyl]oxy]-äthoxy}-1,9b-dihydro-3-isopropyl-3-methyl-2H-imidazo[1',2':1,2]pyrrolo[3,4-b]pyridin-2,5(3H)-dion-Natriumsalz,

1,11b-Dihydro-11b-isopropoxy-3-isopropyl-3-methyl-2H-imidazo[1',2':1,2]pyrrolo[3,4-b]chinolin-2,5(3H)-dion.

Die Verbindungen der Formel I sind, mit Ausnahme von 1,9b-Dihydro-9b-hydroxy-3-isopropyl-3-methyl-2-thioxo-2H-imidazo[1'2':1,2]pyrrolo[3,4-b]pyridin-5(3H)-on (siehe oben) sowie 1,11b-Dihydro-11b-hydroxy-3-isopropyl-3-methyl-2H-imidazo[1',2':1,2]pyrrolo[3,4-b]chinolin-2,5(3H)-dion, d.h. der Verbindung der Formel

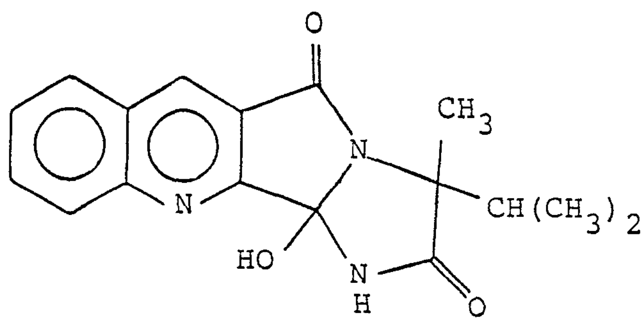

neue Verbindungen. Diese neuen Verbindungen der Formel I sowie das Verfahren zur Herstellung dieser Verbindungen bilden ebenfalls Gegenstand der vorliegenden Erfindung.

Die bekannten Verbindungen und auch deren Herstellung wird auf Seiten 57-59 der Europäischen Patentpublikation Nr. 133.309 bzw. auf Seite 165 der Europäischen Patentpublikation Nr. 41.623 beschrieben. Ueber die Verwendungsmöglichkeit der zweitgenannten Verbindung wird in EP 41.623 nichts ausgesagt.

Das erfindungsgemässe Verfahren zur Herstellung der neuen Verbindungen der allgemeinen Formel I ist dadurch gekennzeichnet, dass man eine Verbindung der allgemeinen Formel

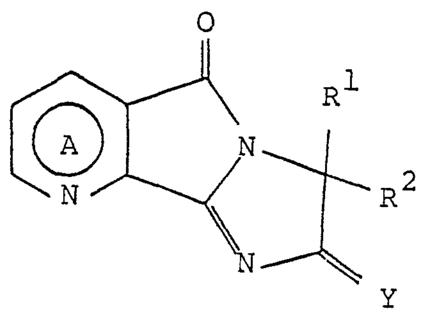

II

worin der Ring A, $R^1$, $R^2$ und Y die oben angegebenen Bedeutungen besitzen,

mit einer Verbindung der allgemeinen Formel

$R^3ZH$ III

worin $R^3$ und Z die oben angegebenen Bedeutungen besitzen,

umsetzt.

Die Umsetzung erfolgt zweckmässigerweise in einem Verdünnungsmittel bei Temperaturen zwischen -20°C und 100°C, vorzugsweise jedoch zwischen 0°C und 40°C. Zudem wird vorteilhaft in Gegenwart eines sauren Katalysators gearbeitet. Als Verdünnungsmittel eignen sich insbesondere organische Lösungsmittel, vorzugsweise aprotische organische Lösungsmittel, wie halogenierte Kohlenwasserstoffe, z.B. Methylenchlorid und Chloroform, und aliphatische oder cyclische Aether, z.B. Diäthyläther und Tetrahydrofuran. Bevorzugte saure Katalysatoren sind organische Säuren, wie Essigsäure, Trifluoressigsäure und p-Toluolsulfonsäure; anorganische Säuren, wie Chlorwasserstoff; Lewissäuren, wie Titantetrachlorid und Aluminiumtrichlorid; polymergebundene Säuren; polymerische Säuren; und Kieselgel.

Die Isolierung und die Reinigung der so hergestellten Verbindungen der Formel I können in an sich bekannter Weise erfolgen.

Die Ausgangsmaterialien der Formel II, in denen Y Sauerstoff bedeutet, sind entweder bekannt oder können nach an sich bekannten Methoden, z.B. gemäss der Europäischen Patentpublikation Nr. 41.623 (siehe insbesondere Seiten 9, 10, 29-33, 35-40, 43, 44, 49, 50, 52-59, 61-64, 74-80, 101-104, 114, 137, 138 und 140-147), hergestellt werden. Die dort beschriebene Methode führt zwar hauptsächlich zum Imidazopyrrolopyridindion der Formel II, jedoch fällt gleichzeitig als Nebenprodukt das entsprechende geometrische Isomere der allgemeinen Formel

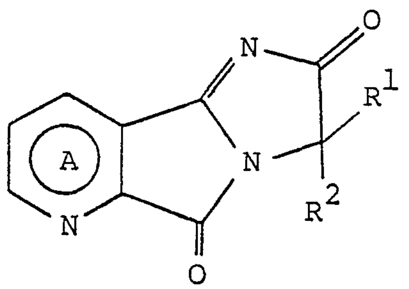

II'

worin der Ring A, $R^1$ und $R^2$ die oben angegebenen Bedeutungen besitzen,

in geringer Menge an, vgl. diesbezüglich Seite 30, Zeile 26 bis Seite 31, Zeile 24, Seite 33 und Seite 78 (Beispiel 3) der EP 41.623. Dieses Nebenprodukt kann auf dieser Stufe in an sich bekannter Weise entfernt werden oder zusammen mit der Verbindung der Formel II, in der Y Sauerstoff bedeutet, mit der Verbindung der Formel III zwecks Herstellung des Endproduktes der Formel I weiter umgesetzt werden. Im letzteren Falle entsteht neben der Verbindung der Formel I in geringer Menge die Verbindung der allgemeinen Formel

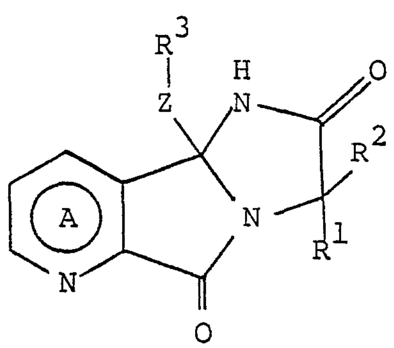

I'

worin der Ring A, $R^1$, $R^2$, $R^3$ und Z die oben angegebenen Bedeutungen besitzen. Das gewünschte Endprodukt der Formel I kann, falls gewünscht, anschliessend in an sich bekannter Weise vom Nebenprodukt der Formel I' befreit werden.

Die Ausgangsmaterialien der Formel II, in denen Y Schwefel bedeuten, können ihrerseits dadurch hergestellt werden, dass man ein Nitril der allgemeinen Formel

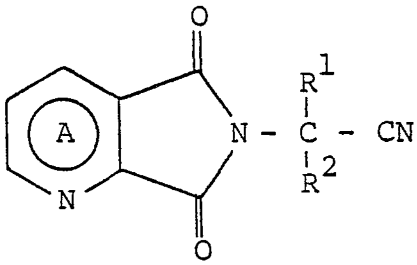

IV

worin der Ring A, $R^1$ und $R^2$ die oben angegebenen Bedeutungen besitzen,

mit gasförmigem Schwefelwasserstoff versetzt, und das daraus resultierende Thioamid der allgemeinen Formel

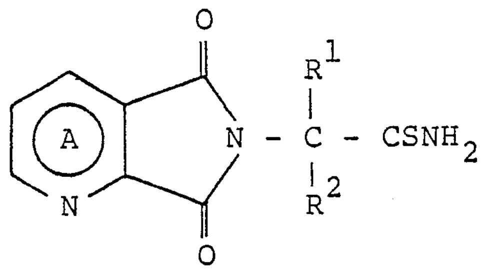

V

einer base- oder säurekatalysierten Cyclisierung unterwirft.

Die Behandlung mit gasförmigem Schwefelwasserstoff erfolgt zweckmässigerweise in einem inerten Verdünnungsmittel bei Temperaturen zwischen 0 und 100°C, vorzugsweise zwischen 0 und 20°C, bis zur Sättigung. Als Verdünnungsmittel eignen sich insbesondere organische aprotische Lösungsmittel, wie halogenierte Kohlenwasserstoffe, z.B. Methylenchlorid und Chloroform, und sekundäre oder tertiäre niedere Alkanole, z.B. Isopropanol und tert.Butanol. Nach Sättigung des Reaktionsgemisches mit Schwefelwasserstoff wird vorteilhaft während 1 bis 7 Tagen stehen gelassen, wonach die Isolierung und die Reinigung des Thioamids der Formel V in an sich bekannter Weise erfolgen können.

Die Cyclisierung des Thioamids zum Ausgangsmaterial der Formel II kann in an sich bekannter Weise durchgeführt werden, z.B. analog der im Beispiel 3 (Seite 78) der Europäischen Patentpublikation Nr. 41.623 beschriebenen Cyclisierung des 5,7-Dihydro-α-isopropyl-α-methyl-5,7-dioxo-6H-pyrrolo[3,4-b]pyridin-6-acetamids. Auch bei der Cyclisierung des Thioamids der Formel V wird üblicherweise ein kleiner Anteil des entsprechenden geometrischen Isomeren der allgemeinen Formel

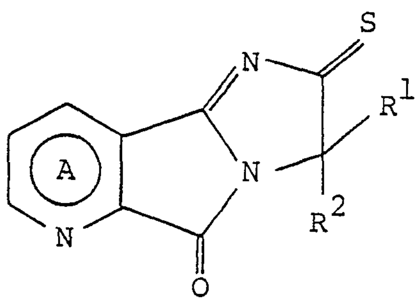

II"

worin der Ring A, $R^1$ und $R^2$ die oben angegebenen Bedeutungen besitzen,

als Nebenprodukt erzeugt. Auch für dieses Nebenprodukt gelten die obigen Ausführungen: Das entsprechende Nebenprodukt von I ist demnach die Verbindung der allgemeinen Formel

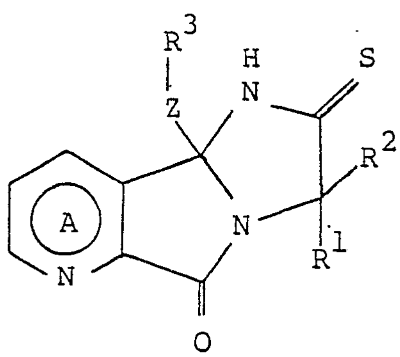

I"

worin der Ring A, $R^1$, $R^2$, $R^3$ und Z die oben angegebenen Bedeutungen besitzen.

Die in dem zweistufigen Verfahren verwendeten Nitrile der Formel IV sind entweder bekannt oder können nach an sich bekannten Methoden hergestellt werden (siehe z.B. die Europäische Patentpublikation Nr. 41.623).

Eine weitere Methode zur Herstellung der Ausgangsmaterialien der Formel II besteht darin, dass man eine Nicotinsäure der allgemeinen Formel

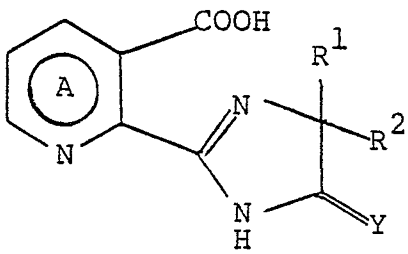

VI

worin der Ring A, $R^1$, $R^2$ und Y die oben angegebenen Bedeutungen besitzen,

einer Cyclisierung unter Wasserabspaltung unterwirft. Für den Fall, dass in der Formel VI Y Sauerstoff bedeutet, erfolgt die Cyclisierung zweckmässigerweise durch Erhitzen der Nicotinsäure VI in einem Gemisch von Essigsäureanhydrid und Essigsäure, das gleichzeitig als Lösungsmittel dient, auf Rückflusstemperatur. Nach Entfernen des Lösungsmittels, z.B. durch Abdampfen unter vermindertem Druck, kann das Produkt nach an sich bekannten Methoden gereinigt werden. Stellt Y der Formel VI hingegen Schwefel dar, werden zweckmässigerweise Trifluoressigsäureanhydrid als wassereliminierendes Mittel sowie ein organisches Lösungsmittels, wie ein aliphatischer chlorierter Kohlenwasserstoff, z.B. Methylenchlorid oder Chloroform, verwendet; in diesem Fall erfolgt die Reaktion bei tiefen Temperaturen, insbesondere im Temperaturbereich -80°C bis -40°C.

Die Nicotinsäuren der Formel VI sind entweder bekannt oder können nach an sich bekannten Methoden hergestellt werden (siehe z.B. die Europäische Patentpublikation Nr. 133.309).

Die Ausgangsmaterialien der Formel III sind entweder bekannt oder können nach an sich bekannten Methoden hergestellt werden.

Die Verbindungen der Formel I besitzen herbizide Eigenschaften und eignen sich besonders zur Bekämpfung von Unkräutern, insbesondere von Hühnerhirse (Echinochloa crus-galli), Grosser Borstenhirse (Setaria faberii), Wehrloser Trespe (Bromus inernis), Gemeiner Quecke (Agropyron repens), Blutfingerhirse (Digitaria sanguinalis), Weissem Gänsefuss (Chenopodium album), Rauhaarigem Amaranth (Amaranthus retroflexus), Ackersenf (Sinapis arvensis), Gemeinem Stechapfel (Datura stramonium), Klettenlabkraut (Galium aparine) und Spitzklette (Xanthium pensylvanicum), in diversen Nutzpflanzenkulturen. Einige Vertreter der Verbindungen I eignen sich als selektive Herbizide in Kulturpflanzen, insbesondere in Soja (Gycine max)-, Mais (Zea mays)- und Weizen (Tritia aestivum)-kulturen, andere als Totalherbizide zur Bekämpfung von Unkräutern nach Getreide- oder Maisernten oder als Herbizide zur Anwendung auf Industriegeländen, Strassenrändern und Wegen.

Im allgemeinen genügt eine Konzentration von 100-1000 g Wirkstoff der Formel I/ha, vorzugsweise 200-500 g Wirkstoff der Formel I/ha, um den gewünschten herbiziden Effekt zu erzielen.

Die Verbindungen der Formel I sind sowohl Vorauflauf-Herbizide als auch Nachauflauf-Herbizide, wobei als letztere die Verbindungen insbesondere auf den Blättern wirksam sind.

Das erfindungsgemässe Unkrautbekämpfungsmittel ist dadurch gekennzeichnet, dass es eine wirksame Menge mindestens einer Verbindung der Formel I, wie oben definiert, sowie Formulierungshilfsstoffe enthält. Das Mittel enthält zweckmässigerweise zumindest einen der folgenden Formulierungshilfsstoffe: feste Trägerstoffe; Lösungs- bzw. Dispersionsmittel; Tenside (Netz- und Emulgiermittel); Dispergatoren (ohne Tensidwirkung); und Stabilisatoren. Unter Verwendung solcher und anderer Hilfsstoffe können die Verbindungen der Formel I, also die herbiziden Wirkstoffe, in die üblichen Formulierungen übergeführt werden, wie Stäube, Pulver, Granulate, Lösungen, Emulsionen, Suspensionen, emulgierbare Konzentrate, Pasten und dergleichen.

Die Verbindungen der Formel I sind im allgemeinen wasserunlöslich und können nach den für wasserunlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln und/oder von Dispergatoren, durch Verdünnen bereits vorbereiteter emulgierbarer Konzentrate mit Lösungs- bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorrillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs- bzw. Dispersionsmittel kommen im wesentlichen in Frage: Aromaten, wie Benzol, Toluol, Xylole und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon

und Dimethylsulfoxid, wobei solche Lösungsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen sein, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäuren, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butylhydroxytoluol; UV-Absorber, z.B. substituierte Benzophenone, Diphenylacrylonitrilsäureester und Zimtsäureester; und Deaktivatoren, z.B. Salze der Aethylendiaminotetraessigsäure und Polyglykole.

Die erfindungsgemässen Unkrautbekämpfungsmittel können zusätzlich zu den Verbindungen der Formel I Synergisten und andere Wirkstoffe, z.B. Insektizide, Akarizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0,01 und 95 Gewichtsprozent, vorzugsweise zwischen 0,5 und 75 Gewichtsprozent einer bzw. mehrerer Verbindungen der Formel I als Wirkstoff(e). Sie können z.B. in einer Form vorliegen, die sich für die Lagerung und den Transport eignet. In solchen Formulierungen, z.B. emulgierbaren Konzentraten, ist die Wirkstoffkonzentration normalerweise im höheren Bereich, vorzugsweise zwischen 1 und 50 Gewichtsprozent, insbesondere zwischen 10 und 20 Gewichtsprozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0,01 bis 10 Gewichtsprozent, insbesondere ca. 0,5 bis 5 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine Verbindung der Formel I, mit festem Trägerstoff vermischt werden, z.B. durch Zusammenmahlen; oder man kann den festen Trägerstoff mit einer Lösung oder Suspension des Wirkstoffes imprägnieren und dann das Lösungs- bzw. Dispersionsmittel durch Abdunsten, Erhitzen oder Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die Verbindung der Formel I kann auch mit einem Tensid und einem festen Trägerstoff zur Bildung eines netzbaren Pulvers vermischt werden, welches in Wasser dispergierbar ist, oder sie kann mit einem festen vorgranulierten Trägerstoff zur Bildung eines granulatförmigen Produktes vermischt werden.

Wenn gewünscht, kann die Verbindung der Formel I in einem mit Wasser nicht mischbaren Lösungsmittel, wie beispielsweise einem hochsiedenden Kohlenwasserstoff, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel, die einen weiteren Gegenstand der vorliegenden Erfindung bildet, kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einer erfindungsgemässen Verbindung der Formel I bzw. einem erfindungsgemässen Unkrautbekämpfungsmittel behandelt.

Die nachfolgenden Beispiele dienen zur näheren Erläuterung der Erfindung.

I. Herstellung der Wirkstoffe der Formel I:

Beispiel 1

Zu einer Lösung von 200 g 3-Isopropyl-3-methyl-2H-imidazo[1',2':1,2]pyrrolo[3,4-b] pyridin -2,5(3H)-dion in 900 ml Isopropylacetat und 100 ml absolutem Isopropanol werden 100 g Kieselgel gegeben, dann wird die resultierende Suspension während ca. 16 Stunden bei Raumtemperatur gerührt. Anschliessend filtriert man das Gemisch, dampft das Filtrat zur Trockene ein und

kristallisiert den Rückstand aus Aethylacetat/n-Hexan. Man erhält das 1,9b-Dihydro-9b-isopropoxy-3-isopropyl-3-methyl -2H-imidazo[1',2':1,2] pyrrolo[3,4-b]pyridin-2,5(3H)-dion als farblose Kristalle, Smp. 158-160°C.

Beispiel 2

Man versetzt eine Lösung von 2 g 3-Isopropyl-3-methyl-2H - imidazo[1',2':1,2]pyrrolo[3,4-b]pyridin-2,5(3H)-dion in 90 ml Methylenchlorid und 10 ml 2-Aethoxyäthanol mit 0,5 ml Trifluoressigsäure und rührt das Reaktionsgemisch 60 Minuten bei Raumtemperatur. Anschliessend wird das Gemisch unter vermindertem Druck zur Trockene eingedampft und der Rückstand aus Aethylacetat/n-Hexan kristallisiert. Man erhält dabei farblose Kristalle, Smp. 115-117C, von 9b-(2-Aethoxyäthoxy)-1,9b-dihydro-3-isopropyl - 3-methyl-2H-imidazo[1',2':1,2]pyrrolo[3,4-b]pyridin - 2,5(3H)-dion.

Beispiele 3-36

Analog dem in Beispiel 1 oder 2 beschriebenen Verfahren werden die entsprechenden Ausgangsmaterialien der Formeln II und III umgesetzt, um die in der nachfolgenden Tabellen 1 und 2 aufgeführten Verbindungen der Formeln Ia bzw. Ib herzustellen.

Tabelle 1

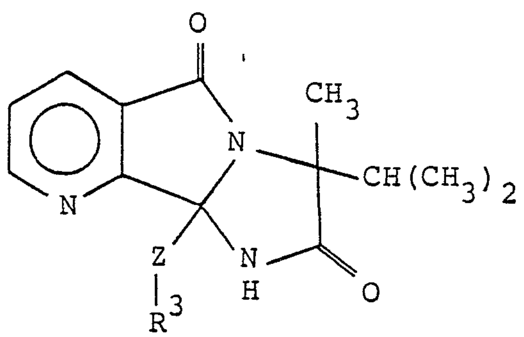

Ia

| Beispiel | $R^3$ | Z | Smp. (°C) |
|---|---|---|---|
| 3 | $-CH(CH_3)_2$ | S | 132-134 |
| 4 | $-CH_2CH(CH_3)(C_2H_5)$ | O | 131-133 |
| 5 | $-CH(CH_3)(C_2H_5)$ | S | 126-128 |
| 6 | $-CH_2-C\equiv CH$ | O | 135-137 |
| 7 | Cyclohexyl | O | 147-148 |
| 8 | $-CH_2CH_2OH$ | O | 130-132 |
| 9 | $-CH_2CH_2NHCOOCH_3$ | O | 130-132 |
| 10 | Cyclopropylmethyl | O | 147-149 |
| 11 | $-CH_2CH_2CN$ | O | 117-119 |
| 12 | $-CH_2CH_2SO_2CH_3$ | O | 132-133 |
| 13 | $-CH_2CH_2SCH_3$ | O | 112-114 |
| 14 | Cyclopentyl | O | 152-154 |
| 15 | $-CH_2C(CH_3)_3$ | O | 177-179 |
| 16 | $-CH_2CH(CH_3)_2$ | O | 137-139 |
| 17 | $-CH_2CH_2OCH_3$ | O | |

Tabelle 2

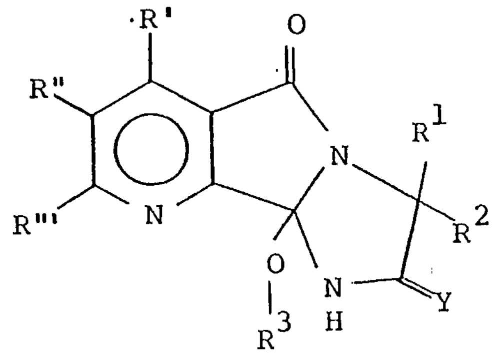

Ib

| Beispiel | R' | R'' | R''' | $R^1$ | $R^2$ | $R^3$ | Y | Smp. (°C) |
|---|---|---|---|---|---|---|---|---|
| 18 | H | H | H | $CH_3$ | Cyclopropyl | Cyclopentyl | O | 109-117 |
| 19 | H | H | H | $CH_3$ | $CH_3$ | Cyclopentyl | O | 142-144 |
| 20 | $CH_3$ | H | H | $CH_3$ | $-CH(CH_3)_2$ | $-CH_2CH_2OC_2H_5$ | O | 141-142 |
| 21 | H | 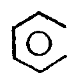 | | $CH_3$ | $-CH(CH_3)_2$ | Cyclopentyl | O | 165 |
| 22 | H | $C_2H_5O$ | H | $CH_3$ | $-CH(CH_3)_2$ | Cyclopentyl | O | |
| 23 | H | H | Cl | $CH_3$ | $-CH(CH_3)_2$ | $-CH_2CH_2OC_2H_5$ | O | |
| 24 | H | $C_2H_5$ | H | $CH_3$ | $-CH(CH_3)_2$ | Cyclopentyl | O | |
| 25 | $CH_3$ | H | H | $CH_3$ | $-CH(CH_3)_2$ | $-CH_2CH_2OCH_3$ | O | |
| 26 | $CH_3$ | H | H | $CH_3$ | $-CH(CH_3)_2$ | $-CH_2CH_2OC_6H_5$ | O | |
| 27 | H | H | H | $-(CH_2)_4-$ | | $-CH_2CH_2OC_2H_5$ | O | |
| 28 | H | H | H | $-(CH_2)_2-$ | | $-CH_2CH_2OC_2H_5$ | O | |
| 29 | H | H | H | $CH_3$ | $-CH(CH_3)_2$ | $-CH_2CH_2OC_2H_5$ | S | |
| 30 | H | $CF_3$ | H | $CH_3$ | $-CH(CH_3)_2$ | $-CH_2CH_2OC_2H_5$ | O | |

Tabelle 2 (Fortsetzung)

| Beispiel | R' | R" | R"' | $R^1$ | $R^2$ | $R^3$ | Y | Smp. (°C) |
|---|---|---|---|---|---|---|---|---|
| 31 | H | $C_2H_5O$ | $NO_2$ | $CH_3$ | $-CH(CH_3)_2$ | $-CH_2CH_2OC_2H_5$ | O | 125 |
| 32 | H | $CH_3OCH_2$ | H | $CH_3$ | $-CH(CH_3)_2$ | $-CH_2CH_2OC_2H_5$ | O | |
| 33 | H | $C_2H_5O$ | $CH_3S$ | $CH_3$ | $-CH(CH_3)_2$ | $-CH_2CH_2OC_2H_5$ | O | |
| 34 | H | $C_2H_5O$ | $C_6H_5S$ | $CH_3$ | $-CH(CH_3)_2$ | $-CH_2CH_2OC_2H_5$ | O | |
| 35 | H | $C_2H_5O$ | 2-Chlor-4-trifluor-methyl-phenoxy | $CH_3$ | $-CH(CH_3)_2$ | $-CH_2CH_2OC_2H_5$ | O | |
| 36 | H | H | H | $-C(CH_3)_2CH_2-$ | | $-CH_2CH_2OC_2H_5$ | O | |

Beispiel 37

Eine Lösung von 2 g 3-Isopropyl-3-methyl-2H-imidazo[1',2':1,2]pyrrolo[3,4-b]pyridin-2,5(3H)-dion in 90 ml Aceton und 10 ml Wasser wird während ca. 16 Stunden bei Raumtemperatur gerührt. Anschliessend dampft man die Lösung zur Trockene ein und kristallisiert den Rückstand aus Aethylacetat/n-Hexan. Man erhält das 1,9b-Dihydro-9b-hydroxy-3-isopropyl-3-methyl-2H-imidazo[1',2':1,2]pyrrolo[3,4-b]pyridin-2,5(3H)-dion als farblose Kristalle, Smp. 151-153°C.

Beispiele 38-40

Analog dem in Beispiel 37 beschriebenen Verfahren werden die entsprechenden Ausgangsmaterialien der Formel II mit Wasser in Aceton behandelt, um die in der nachfolgenden Tabelle 3 aufgeführten Verbindungen der Formel Ic herzustellen.

Tabelle 3

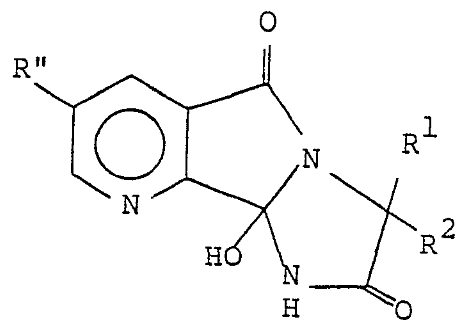

Ic

| Beispiel | R" | $R^1$ | $R^2$ | Smp. (°C) |
|---|---|---|---|---|
| 38 | $C_2H_5$ | $CH_3$ | $-CH(CH_3)_2$ | 146-148 |
| 39 | $C_2H_5O$ | $CH_3$ | $-CH(CH_3)_2$ | 187 |
| 40 | H | $-(CH_2)_4-$ | | 146-151 |

II. Formulierungsbeispiele:

Beispiel 41

Zur Herstellung eines emulgierbaren Konzentrates werden die nachstehend aufgeführten Bestandteile miteinander vermischt, und zwar durch Lösung des Wirkstoffes (eventuell bei erhöhter Temperatur) im Tensid/Lösungsmittel-Gemisch:

| | | |
|---|---|---|
| Verbindung der Formel I (Wirkstoff) | | 125 g |
| Isopropanol | | 125 ml |
| Nonylphenol-(8)-äthoxylat | | 50 g |
| Dodecylbenzolsulfonsäure-Calciumsalz | | 25 g |
| Essigsäure | | 25 ml |
| 1,1,1-Trichloräthan | auf | 1000 ml |

Das so erhaltene klare Konzentrat emulgiert spontan in Wasser. Die sich ergebende Emulsion eignet sich als gebrauchsfertige Spritzbrühe.

Beispiel 42

Zur Herstellung eines suspendierbaren ölhaltigen Konzentrates werden die nachstehend aufgeführten Bestandteile miteinander vermischt und mittels einer Kolloid- oder Kugelmühle möglichst fein vermahlen.

| | Gewichtsprozent |
|---|---|
| Verbindung der Formel I (Wirkstoff) | 25 |
| Nichtiogenes/anionaktives Emulgatorgemisch | 16 |
| Hydratisierte Kieselsäure | 1 |
| Mineralöl-Raffinat | 58 |

Das so erhaltene Konzentrat bildet durch Rühren in Wasser eine homogene Emulsion oder Suspension, die sich als gebrauchsfertige Spritzbrühe eignet.

**Ansprüche**

1. Unkrautbekämpfungsmittel, dadurch gekennzeichnet, dass es eine wirksame Menge mindestens einer Verbindung der allgemeinen Formel

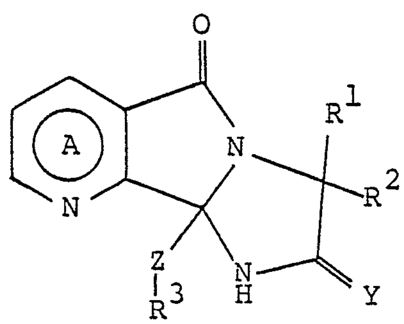

I

worin der

Pyridinring A gegebenenfalls substituiert sein kann, und

$R^1$ gegebenenfalls mit Fluor und/oder Chlor mono- oder mehrfach substituiertes $C_{1-4}$-Alkyl,

$R^2$ $C_{1-10}$-Alkyl oder $C_{3-6}$-Cycloalkyl, oder

$R^1$ und $R^2$ zusammen mit dem Kohlenstoffatom, an das sie geknüpft sind, einen gegebenenfalls mit einem oder zwei $C_{1-4}$-Alkylresten substituierten $C_{3-6}$-Cycloalkanring,

$R^3$ Wasserstoff; unsubstituiertes verzweigtes Alkyl mit bis zu 12 Kohlenstoffatomen; $C_{1-12}$-Alkyl, substituiert mit einem oder mehreren Halogenatomen, einer oder mehreren Hydroxylgruppen, einer Cyanogruppe, einer $C_{3-6}$-Cycloalkylgruppe, einer $C_{1-4}$-Alkoxygruppe, einer Pyridylgruppe, einer gegebenenfalls substituierten Phenoxygruppe, einer α- oder β-Naphthyloxygruppe oder einer der Gruppen (c)-(j)

$-CO-R^6$ (c)

worin

$R^6$ Wasserstoff, Hydroxy, $C_{1-4}$-Alkyl, $C_{1-4}$-Alkoxy oder Phenyl bedeutet,

$-SO_{n'}R^7$ (d)

worin

$R^7$ Hydroxy, Methyl, Phenyl oder p-Tolyl und

n' 0, 1 oder 2 bedeuten,

wobei, falls $R^7$ Hydroxy bedeutet, n' 2 bedeutet,

$-CSO_2R^7$ (e)

worin $R^7$ die oben angegebene Bedeutung besitzt,

einer gegebenenfalls veresterten Phosphit-, Phosphat- oder (f)

Phosphonatgruppe

$-NHCONHR^9$ (g)

worin $R^9$ Wasserstoff, $C_{1-4}$-Alkyl oder Phenyl bedeutet,

$-NHCOOR^{10}$ (h)

worin $R^{10}$ $C_{1-4}$-Alkyl bedeutet,

$-OCONHR^9$ (i)

worin $R^9$ die oben angegebene Bedeutung besitzt,

$-OCO(CH_2)_{n'}COR^{11}$ (j)

worin

$R^{11}$ Hydroxy, $C_{1-4}$-Alkoxy, Phenoxy oder Benzyloxy bedeutet und

n' die oben angegebene Bedeutung besitzt,

$C_{3-10}$-Alkinyl; $C_{3-6}$-Cycloalkyl; oder eine Gruppe (a) oder (b)

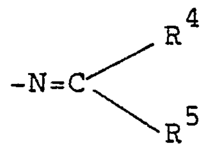
(a)

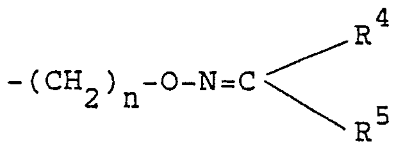
(b)

$R^4$ und $R^5$ unabhängig voneinander $C_{1-4}$-Alkyl,

n 1 oder 2 und

Y und Z unabhängig voneinander Sauerstoff oder Schwefel bedeuten,

mit Ausnahme von 1,9b-Dihydro-9b-hydroxy-3-isopropyl-3-methyl-2-thioxo - 2H-imidazo[1',2':1,2]pyrrolo[3,4-b]pyridin-5(3H)-on

sowie Formulierungshilfsstoffe enthält.

2. Unkrautbekämpfungsmittel nach Anspruch 1, worin $R^1$ der Formel I unsubstituiertes $C_{1-4}$-Alkyl bedeutet.

3. Unkrautbekämpfungsmittel nach Anspruch 2, worin $R^1$ Methyl bedeutet.

4. Unkrautbekämpfungsmittel nach einem der Ansprüche 1 bis 3, worin $R^2$ der Formel I $C_{1-10}$-Alkyl bedeutet.

5. Unkrautbekämpfungsmittel nach Anspruch 4, worin $R^2$ Isopropyl bedeutet.

6. Unkrautbekämpfungsmittel nach einem der Ansprüche 1 bis 5, worin $R^3$ gegebenenfalls substituiertes Alkyl, wie dies in Anspruch 1 näher definiert ist; $C_{3-10}$-Alkinyl; oder $C_{3-6}$-Cycloalkyl bedeutet.

7. Unkrautbekämpfungsmittel nach Anspruch 6, worin $R^3$ als substituiertes Alkyl mit einer oder mehreren Hydroxylgruppen, einer $C_{3-6}$-Cycloalkylgruppe, einer $C_{1-4}$-Alkoxygruppe oder einer gegebenenfalls substituierten Phenoxygruppe substituiertes $C_{1-12}$-Alkyl bedeutet.

8. Unkrautbekämpfungsmittel nach Anspruch 6, worin $R^3$ Isopropyl, Isobutyl, 2-Methoxyäthyl oder 2-Aethoxyäthyl bedeutet.

9. Unkrautbekämpfungsmittel nach Anspruch 1, dadurch gekennzeichnet, dass es als Verbindung der Formel I 1,9b-Dihydro-9b-isopropoxy-3-isopropyl-3-methyl - 2H-imidazo[1',2':1,2]pyrrolo[3,4-b]pyridin-2,5(3H)-dion enthält.

10. Unkrautbekämpfungsmittel nach Anspruch 1, dadurch gekennzeichnet, dass es als Verbindung der Formel I 1,9b-Dihydro-9b-isobutoxy-3-isopropyl-3-methyl - 2H-imidazo[1',2':1,2]pyrrolo[3,4-b]pyridin-2,5(3H)-dion enthält.

11. Unkrautbekämpfungsmittel nach Anspruch 1, dadurch gekennzeichnet, dass es als Verbindung der Formel I 1,9b-Dihydro-3-isopropyl-9b-(2-methoxyäthoxy)-3-methyl - 2H-imidazo[1',2':1,2]pyrrolo[3,4-b]pyridin-2,5(3H)-dion enthält.

12. Unkrautbekämpfungsmittel nach Anspruch 1, dadurch gekennzeichnet, dass es als Verbindung der Formel I 9b-(2-Aethoxyäthoxy)-1,9b-dihydro-3-isopropyl-3-methyl-2H-imidazo[1',2':1,2]pyrrolo[3,4-b]pyridin-2,5(3H)-dion enthält.

13. Verbindungen der allgemeinen Formel

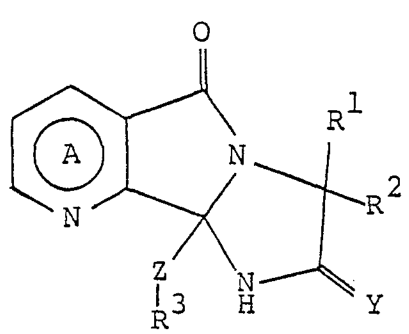
I

worin der

Pyridinring A gegebenenfalls substituiert sein kann, und

$R^1$ gegebenenfalls mit Fluor und/oder Chlor mono- oder mehrfach substituiertes $C_{1-4}$-Alkyl,

$R^2$ $C_{1-10}$-Alkyl oder $C_{3-6}$-Cycloalkyl, oder

$R^1$ und $R^2$ zusammen mit dem Kohlenstoffatom, an das sie geknüpft sind, einen gegebenenfalls mit einem oder zwei $C_{1-4}$-Alkylresten substituierten $C_{3-6}$-Cycloalkanring,

$R^3$ Wasserstoff; unsubstituiertes verzweigtes Alkyl mit bis zu 12 Kohlenstoffatomen; $C_{1-12}$-Alkyl, substituiert mit einem oder mehreren Halogenatomen, einer oder mehreren Hydroxylgruppen, einer Cyanogruppe, einer $C_{3-6}$-Cycloalkylgruppe, einer $C_{1-4}$-Alkoxygruppe, einer Pyridylgruppe, einer gegebenenfalls substituierten Phenoxygruppe, einer $\alpha$- oder $\beta$-Naphthyloxygruppe oder einer der Gruppen (c)-(j)

$-CO-R^6$ (c)

worin

$R^6$ Wasserstoff, Hydroxy, $C_{1-4}$-Alkyl, $C_{1-4}$-Alkoxy oder Phenyl bedeutet,

$-SO_{n'}R^7$ (d)

worin

$R^7$ Hydroxy, Methyl, Phenyl oder p-Tolyl und

n' 0, 1 oder 2 bedeuten,

wobei, falls $R^7$ Hydroxy bedeutet, n' 2 bedeutet,

$-OSO_2R^7$ (e)

worin $R^7$ die oben angegebene Bedeutung besitzt,

einer gegebenenfalls veresterten Phosphit-, Phosphat- oder (f)

Phosphonatgruppe

$-NHCONHR^9$ (g)

worin $R^9$ Wasserstoff, $C_{1-4}$-Alkyl oder Phenyl bedeutet,

$-NHCOOR^{10}$ (h)

worin $R^{10}$ $C_{1-4}$-Alkyl bedeutet,

$-OCONHR^9$ (i)

worin $R^9$ die oben angegebene Bedeutung besitzt,

$-OCO(CH_2)_{n'}COR^{11}$ (j)

worin

$R^{11}$ Hydroxy, $C_{1-4}$-Alkoxy, Phenoxy oder Benzyloxy bedeutet und

n' die oben angegebene Bedeutung besitzt,

$C_{3-10}$-Alkinyl; $C_{3-6}$-Cycloalkyl; oder eine Gruppe (a) oder (b)

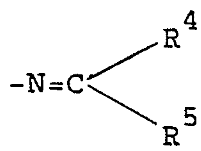

(a)

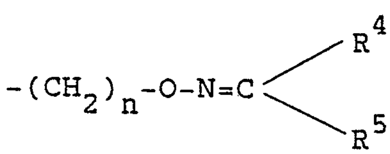

(b)

$R^4$ und $R^5$ unabhängig voneinander $C_{1-4}$-Alkyl,

n 1 oder 2 und

Y und Z unabhängig voneinander Sauerstoff oder Schwefel bedeuten,

mit Ausnahme von 1,9b-Dihydro-9b-hydroxy-3-isopropyl-3-methyl-2-thioxo-2H-imidazo[1',2':1,2]pyrrolo[3,4-b]pyridin-5(3H)-on sowie von 1,11b-Dihydro-11b-hydroxy-3-isopropyl-3-methyl-2H-imidazo[1',2':1,2]pyrrolo[3,4-b]chinolin-2,5(3H)-dion.

14. Verbindungen nach Anspruch 13, worin $R^1$ unsubstituiertes $C_{1-4}$-Alkyl bedeutet.

15. Verbindungen nach Anspruch 14, worin $R^1$ Methyl bedeutet.

16. Verbindungen nach einem der Ansprüche 13 bis 15, worin $R^2$ $C_{1-10}$-Alkyl bedeutet.

17. Verbindungen nach Anspruch 16, worin $R^2$ Isopropyl bedeutet.

18. Verbindungen nach einem der Ansprüche 13 bis 17, worin $R^3$ gegebenenfalls substituiertes Alkyl, wie dies in Anspruch 13 näher definiert ist; $C_{3-10}$-Alkinyl; oder $C_{3-6}$-Cycloalkyl bedeutet.

19. Verbindungen nach Anspruch 18, worin $R^3$ als substituiertes Alkyl mit einer oder mehreren Hydroxylgruppen,

einer $C_{3-6}$-Cycloalkylgruppe, einer $C_{1-4}$-Alkoxygruppe oder einer gegebenenfalls substituierten Phenoxygruppe substituiertes $C_{1-12}$-Alkyl bedeutet.

20. Verbindungen nach Anspruch 18, worin $R^3$ Isopropyl, Isobutyl, 2-Methoxyäthyl oder 2-Aethoxyäthyl bedeutet.

21. 1,9b-Dihydro-9b-isopropoxy-3-isopropyl-3-methyl-2H-imidazo[1',2':1,2]pyrrolo[3,4-b]pyridin-2,5(3H)-dion.

22. 1,9b-Dihydro-9b-isobutoxy-3-isopropyl-3-methyl-2H-imidazo-[1',2':1,2]pyrrolo[3,4-b]pyridin-2,5(3H)-dion.

23. 1,9b-Dihydro-3-isopropyl-9b-(2-methoxyäthoxy)-3-methyl-2-H-imidazo[1',2':1,2]pyrrolo[3,4-b]pyridin-2,5(3H)-dion.

24. 9b-(2-Aethoxyäthoxy)-1,9b-dihydro-3-isopropyl-3-methyl-2H--imidazo[1',2':1,2]pyrrolo[3,4-b]pyridin-2,5(3H)-dion.

25. Eine Verbindung nach Anspruch 13, ausgewählt aus:

1,9b-Dihydro-3-isopropyl-9b-isopropylthio-3-methyl-2H-imidazo[1',2':1,2]pyrrolo[3,4-b]pyridin-2,5(3H)-dion,

1,9b-Dihydro-3-isopropyl-3-methyl-9b-(2-methylbutoxy)-2H--imidazo[1',2':1,2]pyrrolo[3,4-b]pyridin-2,5(3H)-dion,

9b-(sek.Butylthio)-1,9b-dihydro-3-isopropyl-3-methyl-2H-imidazo[1',2':1,2]pyrrolo[3,4-b]pyridin-2,5(3H)-dion,

1,9b-Dihydro-3-isopropyl-3-methyl-9b-propargyloxy-2H-imidazo[1',2':1,2]pyrrolo[3,4-b]pyridin-2,5(3H)-dion,

9b-Cyclohexyloxy-1,9b-dihydro-3-isopropyl-3-methyl-2H-imidazo[1',2':1,2]pyrrolo[3,4-b]pyridin-2,5(3H)-dion,

1,9b-Dihydro-9b-(2-hydroxyäthoxy)-3-isopropyl-3-methyl-2-H-imidazo[1',2':1,2]pyrrolo[3,4-b]pyridin-2,5(3H)-dion,

1,9b-Dihydro-3-isopropyl-9b-(2-methoxycarbonylaminoäthoxy)-3-methyl-2H-imidazo[1',2':1,2]pyrrolo[3,4-b]pyridin-2,5(3-H)-dion,

9b-Cyclopropylmethoxy-1,9b-dihydro-3-isopropyl-3-methyl--2H-imidazo[1',2':1,2]pyrrolo[3,4-b]pyridin-2,5(3H)-dion,

9b-(2-Cyanoäthoxy)-1,9b-dihydro-3-isopropyl-3-methyl-2H-imidazo[1',2':1,2]pyrrolo[3,4-b]pyridin-2,5(3H)-dion,

1,9b-Dihydro-3-isopropyl-3-methyl-9b-(2-methylsulfonyläthoxy)-2H-imidazo[1',2':1,2]pyrrolo[3,4-b]pyridin-2,5(3H)-dion,

1,9b-Dihydro-3-isopropyl-3-methyl-9b-(2-methylthioäthoxy)--2H-imidazo[1',2':1,2]pyrrolo[3,4-b]pyridin-2,5(3H)-dion,

9b-Cyclopentyloxy-1,9b-dihydro-3-isopropyl-3-methyl-2H-imidazo[1',2':1,2]pyrrolo[3,4-b]pyridin-2,5(3H)-dion,

1,9b-Dihydro-3-isopropyl-3-methyl-9b-neopentyl-2H-imidazo[1',2':1,2]pyrrolo[3,4-b]pyridin-2,5(3H)-dion und

1,9b-Dihydro-9b-hydroxy-3-isopropyl-3-methyl-2H-imidazo-[1',2':1,2]pyrrolo[3,4-b]pyridin-2,5(3H)-dion.

26. Eine Verbindung nach Anspruch 13, ausgewählt aus:

9b-Cyclopentyloxy-3-cyclopropyl-1,9b-dihydro-3-methyl-2H--imidazo[1',2':1,2]pyrrolo[3,4-b]pyridin-2,5(3H)-dion,

9b-Cyclopentyloxy-1,9b-dihydro-3,3-dimethyl-2H-imidazo[1'-,2':1,2]pyrrolo[3,4-b]pyridin-2,5(3H)-dion,

9b-Aethoxyäthoxy-1,9b-dihydro-3,6-dimethyl-3-isopropyl-2H--imidazo[1',2':1,2]pyrrolo[3,4-b]pyridin-2,5(3H)-dion,

11b-Cyclopentyloxy-1,11b-dihydro-3-isopropyl-3-methyl-2H--imidazo[1',2':1,2]pyrrolo[3,4-b]chinolin-2,5(3H)-dion,

7-Aethoxy-9b-cyclopentyloxy-1,9b-dihydro-3-isopropyl-3-methyl-2H-imidazo[1',2':1,2]pyrrolo[3,4-b]pyridin-2,5(3H)-dion,

9b-(2-Aethoxyäthoxy)-8-chlor-1,9b-dihydro-3-isopropyl-3-methyl-2H-imidazo[1',2':1,2]pyrrolo[3,4-b]pyridin-2,5(3H)-dion,

7-Aethyl-9b-cyclopentyloxy-1,9b-dihydro-3-isopropyl-3-methyl-2H-imidazo[1',2':1,2]pyrrolo[3,4-b]pyridin-2,5(3H)-dion,

1,9b-Dihydro-3,6-dimethyl-3-isopropyl-9b-(2-methoxyäthoxy-)-2H-imidazo[1',2':1,2]pyrrolo[3,4-b]pyridin-2,5(3H)-dion,

1,9b-Dihydro-3,6-dimethyl-3-isopropyl-9b-(2-phenoxyäthyl)--2H-imidazo[1',2':1,2]pyrrolo[3,4-b]pyridin-2,5(3H)-dion,

9'b-(2-Aethoxyäthoxy)-1',9'b-dihydro-spirocyclopentan-1,3'(-5'H)imidazo[1',2':1,2]pyrrolo[3,4-b]pyridin-2',5'(3'H)-dion,

9'b-(2-Aethoxyäthoxy)-1',9'b-dihydro-spirocyclopropan-1,3'(-5'H)imidazo[1',2':1,2]pyrrolo[3,4-b]pyridin-2',5'(3'H)-dion,

9b-(2-Aethoxyäthoxy)-1,9b-dihydro-3-isopropyl-3-methyl-2-thioxo-2H-imidazo[1',2':1,2]pyrrolo[3,4-b]pyridin-5(3H)-on,

9b-(2-Aethoxyäthoxy)-1,9b-dihydro-3-isopropyl-3-methyl-7-trifluormethyl-2H-imidazo[1',2':1,2]pyrrolo[3,4-b]pyridin-2,5(-3h)-dion,

7-Aethoxy-9b-(2-äthoxyäthoxy)-1,9b-dihydro-3-isopropyl-3--methyl-8-nitro-2H-imidazo[1',2':1,2]pyrrolo[3,4-b]pyridin-2,5-(3H)-dion,

9b-(2-Aethoxyäthoxy)-1,9b-dihydro-3-isopropyl-7-(methoxymethyl)-3-methyl-2H-imidazo[1',2':1,2]pyrrolo[3,4-b]pyridin--2,5(3H)-dion,

7-Aethoxy-9b-(2-äthoxyäthoxy)-1,9b-dihydro-3-isopropyl-3--methyl-8-methylthio-2H-imidazo[1',2':1,2]pyrrolo[3,4-b]pyridin-2,5(3H)-dion,

7-Aethoxy-9b-(2-äthoxyäthoxy)-1,9b-dihydro-3-isopropyl-3--methyl-8-phenylthio-2H-imidazo[1',2':1,2]pyrrolo[3,4-b]pyridin-2,5(3H)-dion,

7-Aethoxy-9b-(2-äthoxyäthoxy)-8-(2-chlor-4-trifluormethyl-phenoxy)-1,9b-dihydro-3-isopropyl-3-methyl-2H-imidazo[1',2'-:1,2]pyrrolo[3,4-b]pyridin-2,5(3H)-dion,

9'b-(2-Aethoxyäthoxy)-1',9'b-dihydro-spiro(2,2-dimethyl-cyclopropan)-1,3'(5'H)-imidazo[1',2':1,2]pyrrolo[3,4-b]pyridin-2-',5'(3'H)-dion,

7-Aethyl-1,9b-dihydro-9b-hydroxy-3-isopropyl-3-methyl-2H--imidazo[1',2':1,2]pyrrolo[3,4-b]pyridin-2,5(3H)-dion,

7-Aethoxy-1,9b-dihydro-9b-hydroxy-3-isopropyl-3-methyl-2-H-imidazo[1',2':1,2]pyrrolo[3,4-b]pyridin-2,5(3H)-dion und

1',9'b-Dihydro-9'b-hydroxy-spirocyclopentan-1,3'-[2H]-imidazo[1',2':1,2]pyrrolo[3,4-b]pyridin-2,5(3H)-dion.

27. Verbindungen nach Anspruch 13 als Wirkstoffe von Unkrautbekämpfungsmitteln.

28. Verfahren zur Herstellung von Verbindungen der allgemeinen Formel

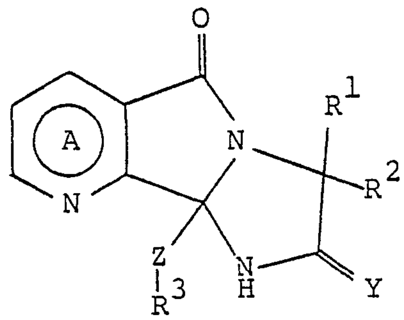

I

worin der

Pyridinring A gegebenenfalls substituiert sein kann, und

$R^1$ gegebenenfalls mit Fluor und/oder Chlor mono- oder mehrfach substituiertes $C_{1-4}$-Alkyl,

$R^2$ $C_{1-10}$-Alkyl oder $C_{3-6}$ -Cycloalkyl, oder

$R^1$ und $R^2$ zusammen mit dem Kohlenstoffatom, an das sie geknüpft sind, einen gegebenenfalls mit einem oder zwei $C_{1-4}$ -Alkylresten substituierten $C_{3-6}$-Cycloalkanring,

$R^3$ Wasserstoff; unsubstituiertes verzweigtes Alkyl mit bis zu 12 Kohlenstoffatomen; $C_{1-12}$-Alkyl, substituiert mit einem oder mehreren Halogenatomen, einer oder mehreren Hydroxylgruppen, einer Cyanogruppe, einer $C_{3-6}$-Cycloalkylgruppe, einer

$C_{1-4}$-Alkoxygruppe, einer Pyridylgruppe, einer gegebenenfalls substituierten Phenoxygruppe, einer $\alpha$- oder $\beta$ -Naphthyloxygruppe oder einer der Gruppen (c)-(j)

$-CO-R^6$ (c)

worin

$R^6$ Wasserstoff, Hydroxy, $C_{1-4}$-Alkyl, $C_{1-4}$-Alkoxy oder Phenyl bedeutet,

$-SO_{n'}R^7$ (d)

worin

$R^7$ Hydroxy, Methyl, Phenyl oder p-Tolyl und

n' 0, 1 oder 2 bedeuten,

wobei, falls $R^7$ Hydroxy bedeutet, n' 2 bedeutet,

$-OSO_2R^7$ (e)

worin $R^7$ die oben angegebene Bedeutung besitzt,

einer gegebenenfalls veresterten Phosphit-, Phosphat- oder (f)

Phosphonatgruppe

$-NHCONHR^9$ (g)

worin $R^9$ Wasserstoff, $C_{1-4}$-Alkyl oder Phenyl bedeutet,

$-NHPCOOR^{10}$ (h)

worin $R^{10}$ $C_{1-4}$-Alkyl bedeutet,

$-OCONHR^9$ (i)

worin $R^9$ die oben angegebene Bedeutung besitzt,

$-OCO(CH_2)_{n'}COR^{11}$ (j)

worin

$R^{11}$ Hydroxy, $C_{1-4}$-Alkoxy, Phenoxy oder Benzyloxy bedeutet und

n' die oben angegebene Bedeutung besitzt,

$C_{3-10}$-Alkinyl; $C_{3-6}$ -Cycloalkyl; oder eine Gruppe (a) oder (b)

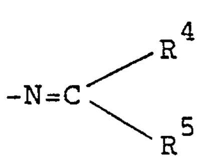

(a)

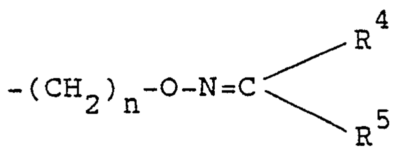

(b)

$R^4$ und $R^5$ unabhängig voneinander $C_{1-4}$-Alkyl,

*n* 1 oder 2 und

Y und Z unabhängig voneinander Sauerstoff oder Schwefel bedeuten,

mit Ausnahme von 1,9b-Dihydro-9b-hydroxy-3-isopropyl-3-methyl-2-thioxo-2H-imidazo[1',2':1,2]pyrrolo[3,4-b]pyridin-5(3H)-on sowie von 1,11b-Dihydro-11b-hydroxy-3-isopropyl-3-methyl-2H-imidazo[1',2':1,2]pyrrolo[3,4-b]chinolin-2,5(3H)-dion, dadurch gekennzeichnet, dass man eine Verbindung der allgemeinen Formel

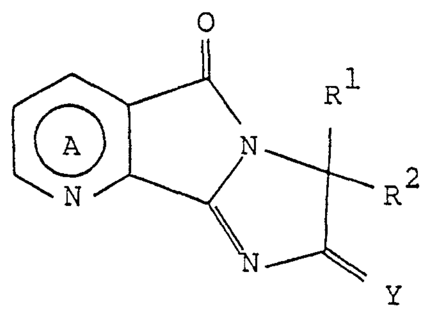

II

worin der Ring A, $R^1$, $R^2$ und Y die oben angegebenen Bedeutungen besitzen,

mit einer Verbindung der allgemeinen Formel

$R^3ZH$ III

worin $R^3$ und Z die oben angegebenen Bedeutungen besitzen,

umsetzt

29. Verfahren zur Bekämpfung von Unkräutern, dadurch gekennzeichnet, dass man das gegen Unkräuter zu schützende Gut und/oder die Unkräuter mit einer wirksamen Menge eines Mittels gemäss einem der Ansprüche 1 bis 6, 9 und 12 bzw. einer Verbindung gemäss einem der Ansprüche 13 bis 18, 21, 24 und 25 behandelt.

30. Verfahren zur Bekämpfung von Unkräutern, dadurch gekennzeichnet, dass man das gegen Unkräuter zu schützende Gut und/oder die Unkräuter mit einer wirksamen Menge eines Mittels gemäss einem der Ansprüche 7, 8, 10 und 11 bzw. einer Verbindung gemäss einem der Ansprüche 19, 20, 22, 23 und 26 behandelt.

31. Verwendung eines Mittels gemäss einem der Ansprüche 1 bis 6, 9 und 12 bzw. einer Verbindung gemäss einem der Ansprüche 13 bis 18, 21, 24 und 25 zur Bekämpfung von Unkräutern.

32. Verwendung eines Mittels gemäss einem der Ansprüche 7, 8, 10 und 11 bzw. einer Verbindung gemäss einem der Ansprüche 19, 20, 22, 23 und 26 zur Bekämpfung von Unkräutern.

Patentansprüche für den Vertragsstaat : AT

1. Unkrautbekämpfungsmittel, dadurch gekennzeichnet, dass es eine wirksame Menge mindestens einer Verbindung der allgemeinen Formel

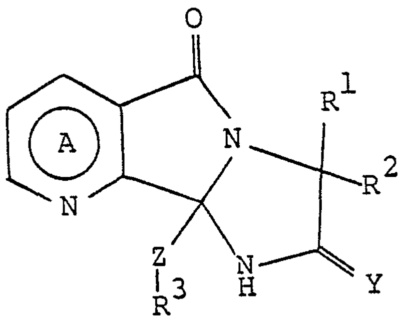

I

worin der

Pyridinring A gegebenenfalls substituiert sein kann, und

$R^1$ gegebenenfalls mit Fluor und/oder Chlor mono- oder mehrfach substituiertes $C_{1-4}$-Alkyl,

$R^2$ $C_{1-10}$-Alkyl oder $C_{3-6}$-Cycloalkyl, oder

$R^1$ und $R^2$ zusammen mit dem Kohlenstoffatom, an das sie geknüpft sind, einen gegebenenfalls mit einem oder zwei $C_{1-4}$-Alkylresten substituierten $C_{3-6}$-Cycloalkanring,

$R^3$ Wasserstoff; unsubstituiertes verzweigtes Alkyl mit bis zu 12 Kohlenstoffatomen; $C_{1-12}$-Alkyl, substituiert mit einem oder mehreren Halogenatomen, einer oder mehreren Hydroxylgruppen, einer Cyanogruppe, einer $C_{3-6}$-Cycloalkylgruppe, einer $C_{1-4}$-Alkoxygruppe, einer Pyridylgruppe, einer gegebenenfalls substituierten Phenoxygruppe, einer α- oder β-Naphthyloxygruppe oder einer der Gruppen (c)-(j)

$-CO-R^6$ (c)

worin

$R^6$ Wasserstoff, Hydroxy, $C_{1-4}$-Alkyl, $C_{1-4}$-Alkoxy oder Phenyl bedeutet,

$-SO_{n'}R^7$ (d)

worin

$R^7$ Hydroxy, Methyl, Phenyl oder p-Tolyl und n' 0, 1 oder 2 bedeuten,

wobei, falls $R^7$ Hydroxy bedeutet, n' 2 bedeutet,

$-OSO_2R^7$ (e)

worin $R^7$ die oben angegebene Bedeutung besitzt,

einer gegebenenfalls veresterten Phosphit-, Phosphat- oder (f)

Phosphonatgruppe

$-NHCONHR^9$ (g)

worin $R^9$ Wasserstoff, $C_{1-4}$-Alkyl oder Phenyl bedeutet,

$-NHCOOR^{10}$ (h)

worin $R^{10}$ $C_{1-4}$-Alkyl bedeutet,

$-OCONHR^9$ (i)

worin $R^9$ die oben angegebene Bedeutung besitzt,

$-OCO(CH_2)_{n'}COR^{11}$ (j)

worin

$R^{11}$ Hydroxy, $C_{1-4}$-Alkoxy, Phenoxy oder Benzyloxy bedeutet und

n' die oben angegebene Bedeutung besitzt,

$C_{3-10}$-Alkinyl; $C_{3-6}$-Cycloalkyl; oder eine Gruppe (a) oder (b)

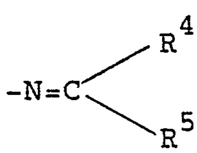

(a)

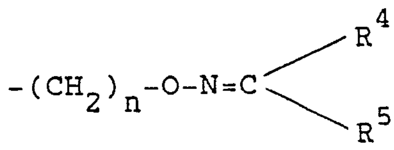 (b)

$R^4$ und $R^5$ unabhängig voneinander $C_{1-4}$-Alkyl,

*n* 1 oder 2 und

Y und Z unabhängig voneinander Sauerstoff oder Schwefel bedeuten,

mit Ausnahme von 1,9b-Dihydro-9b-hydroxy-3-isopropyl-3-methyl-2-thioxo-2H-imidazo[1',2':1,2]pyrrolo[3,4-b]pyridin-5(3H)-on

sowie Formulierungshilfsstoffe enthält.

2. Unkrautbekämpfungsmittel nach Anspruch 1, worin $R^1$ der Formel I unsubstituiertes $C_{1-4}$-Alkyl bedeutet.

3. Unkrautbekämpfungsmittel nach Anspruch 2, worin $R^1$ Methyl bedeutet.

4. Unkrautbekämpfungsmittel nach einem der Ansprüche 1 bis 3, worin $R^2$ der Formel I $C_{1-10}$-Alkyl bedeutet.

5. Unkrautbekämpfungsmittel nach Anspruch 4, worin $R^2$ Isopropyl bedeutet.

6. Unkrautbekämpfungsmittel nach einem der Ansprüche 1 bis 5, worin $R^3$ gegebenenfalls substituiertes Alkyl, wie dies in Anspruch 1 näher definiert ist; $C_{3-10}$-Alkinyl; oder $C_{3-6}$-Cycloalkyl bedeutet.

7. Unkrautbekämpfungsmittel nach Anspruch 6, worin $R^3$ als substituiertes Alkyl mit einer oder mehreren Hydroxylgruppen, einer $C_{3-6}$-Cycloalkylgruppe, einer $C_{1-4}$-Alkoxygruppe oder einer gegebenenfalls substituierten Phenoxygruppe substituiertes $C_{1-12}$-Alkyl bedeutet.

8. Unkrautbekämpfungsmittel nach Anspruch 6, worin $R^3$ Isopropyl, Isobutyl, 2-Methoxyäthyl oder 2-Aethoxyäthyl bedeutet.

9. Unkrautbekämpfungsmittel nach Anspruch 1, dadurch gekennzeichnet, dass es als Verbindung der Formel I 1,9b-Dihydro-9b-isopropoxy-3-isopropyl-3-methyl-2H-imidazo[1',2':1,2]pyrrolo[3,4-b]pyridin-2,5(3H)-dion enthält.

10. Unkrautbekämpfungsmittel nach Anspruch 1, dadurch gekennzeichnet, dass es als Verbindung der Formel I 1,9b-Dihydro-9b-isobutoxy-3-isopropyl-3-methyl-2H-imidazo[1',2':1,2]pyrrolo[3,4-b]pyridin-2,5(3H)-dion enthält.

11. Unkrautbekämpfungsmittel nach Anspruch 1, dadurch gekennzeichnet, dass es als Verbindung der Formel I 1,9b-Dihydro-3-isopropyl-9b-(2-methoxyäthoxy)-3-methyl-2H-imidazo[1',2':1,2]pyrrolo[3,4-b]pyridin-2,5(3H)-dion enthält.

12. Unkrautbekämpfungsmittel nach Anspruch 1, dadurch gekennzeichnet, dass es als Verbindung der Formel I 9b-(2-Aethoxyäthoxy)-1,9b-dihydro-3-isopropyl-3-methyl-2H-imidazo[1',2':1,2]pyrrolo[3,4-b]pyridin-2,5(3H)-dion enthält.

13. Unkrautbekämpfungsmittel nach Anspruch 1, dadurch gekennzeichnet, dass es eine wirksame Menge mindestens einer aus der Gruppe

1,9b-Dihydro-3-isopropyl-9b-isopropylthio-3-methyl-2H-imidazo[1',2':1,2]pyrrolo[3,4-b]pyridin-2,5(3H)-dion,

9b-Cyclopentyloxy-3-cyclopropyl-1,9b-dihydro-3-methyl-2H-imidazo[1',2':1,2]pyrrolo[3,4-b]pyridin-2,5(3H)-dion,

9b-Cyclopentyloxy-1,9b-dihydro-3,3-dimethyl-2H-imidazo[1',2':1,2]pyrrolo[3,4-b]pyridin-2,5(3H)-dion,

9b-Aethoxyäthoxy-1,9b-dihydro-3,6-dimethyl-3-isopropyl-2H-imidazo[1',2':1,2]pyrrolo[3,4-b]pyridin-2,5(3H)-dion,

11b-Cyclopentyloxy-1,11b-dihydro-3-isopropyl-3-methyl-2H-imidazo[1',2':1,2]pyrrolo[3,4-b]chinolin-2,5(3H)-dion,

7-Aethoxy-9b-cyclopentyloxy-1,9b-dihydro-3-isopropyl-3-methyl-2H-imidazo[1',2':1,2]pyrrolo[3,4-b]pyridin-2,5(3H)-dion,

9b-(2-Aethoxyäthoxy)-8-chlor-1,9b-dihydro-3-isopropyl-3-methyl-2H-imidazo[1',2':1,2]pyrrolo[3,4-b]pyridin-2,5(3H)-dion,

7-Aethyl-9b-cyclopentyloxy-1,9b-dihydro-3-isopropyl-3-methyl-2H-imidazo[1',2':1,2]pyrrolo[3,4-b]pyridin-2,5(3H)-dion,

1,9b-Dihydro-3,6-dimethyl-3-isopropyl-9b-(2-methoxyäthoxy)-2H-imidazo[1',2':1,2]pyrrolo[3,4-b]pyridin-2,5(3H)-dion,

1,9b-Dihydro-3,6-dimethyl-3-isopropyl-9b-(2-phenoxyäthyl)-2H-imidazo[1',2':1,2]pyrrolo[3,4-b]pyridin-2,5(3H)-dion,

9'b-(2-Aethoxyäthoxy)-1',9'b-dihydro-spirocyclopentan-1,3'(5'H)imidazo[1',2':1,2]pyrrolo[3,4-b]pyridin-2',5'(3'H)-dion,

9'b-(2-Aethoxyäthoxy)-1',9'b-dihydro-spirocyclopropan-1,3'(5'H)imidazo[1',2':1,2]pyrrolo[3,4-b]pyridin-2',5'(3'H)-dion,

9b-(2-Aethoxyäthoxy)-1,9b-dihydro-3-isopropyl-3-methyl-2-thioxo-2H-imidazo[1',2':1,2]pyrrolo[3,4-b]pyridin-5(3H)-on,

9b-(2-Aethoxyäthoxy)-1,9b-dihydro-3-isopropyl-3-methyl-7-trifluormethyl-2H-imidazo[1',2':1,2]pyrrolo[3,4-b]pyridin-2,5(-3H)-dion,

7-Aethoxy-9b-(2-äthoxyäthoxy)-1,9b-dihydro-3-isopropyl-3--methyl-8-nitro-2H-imidazo[1',2':1,2]pyrrolo[3,4-b]pyridin-2,5-(3H)-dion,

1,9b-Dihydro-3-isopropyl-3-methyl-9b-(2-methylbutoxy)-2H--imidazo[1',2':1,2]pyrrolo[3,4-b]pyridin-2,5(3H)-dion,

9b-(sek.Butylthio)-1,9b-dihydro-3-isopropyl-3-methyl-2H-imidazo[1',2':1,2]pyrrolo[3,4-b]pyridin-2,5(3H)-dion,

1,9b-Dihydro-3-isopropyl-3-methyl-9b-propargyloxy-2H-imidazo[1',2':1,2]pyrrolo[3,4-b]pyridin-2,5(3H)-dion,

9b-Cyclohexyloxy-1,9b-dihydro-3-isopropyl-3-methyl-2H-imidazo[1',2':1,2]pyrrolo[3,4-b]pyridin-2,5(3H)-dion,

1,9b-Dihydro-9b-(2-hydroxyäthoxy)-3-isopropyl-3-methyl-2-H-imidazo[1',2':1,2]pyrrolo[3,4-b]pyridin-2,5(3H)-dion,

1,9b-Dihydro-3-isopropyl-9b-(2-methoxycarbonylaminoäthoxy)-3-methyl-2H-imidazo[1',2':1,2]pyrrolo[3,4-b]pyridin-2,5(3-H)-dion,

9b-Cyclopropylmethoxy-1,9b-dihydro-3-isopropyl-3-methyl--2H-imidazo[1',2':1,2]pyrrolo[3,4-b]pyridin-2,5(3H)-dion,

9b-(2-Cyanoäthoxy)-1,9b-dihydro-3-isopropyl-3-methyl-2H-imidazo[1',2':1,2]pyrrolo[3,4-b]pyridin-2,5(3H)-dion,

1,9b-Dihydro-3-isopropyl-3-methyl-9b-(2-methylsulfonyläthoxy)-2H-imidazo[1',2':1,2]pyrrolo[3,4-b]pyridin-2,5(3H)-dion,

1,9b-Dihydro-3-isopropyl-3-methyl-9b-(2-methylthioäthoxy)--2H-imidazo[1',2':1,2]pyrrolo[3,4-b]pyridin-2,5(3H)-dion,

9b-Cyclopentyloxy-1,9b-dihydro-3-isopropyl-3-methyl-2H-imidazo[1',2':1,2]pyrrolo[3,4-b]pyridin-2,5(3H)-dion,

1,9b-Dihydro-3-isopropyl-3-methyl-9b-neopentyl-2H-imidazo[1',2':1,2]pyrrolo[3,4-b]pyridin-2,5(3H)-dion und

1,9b-Dihydro-9b-hydroxy-3-isopropyl-3-methyl-2H-imidazo[-1',2':1,2]pyrrolo[3,4-b]pyridin-2,5(3H)-dion ausgewählten Verbindung sowie Formulierungshilfsstoffe enthält.

14. Unkrautbekämpfungsmittel nach Anspruch 1, dadurch gekennzeichnet, dass es eine wirksame Menge mindestens einer aus der Gruppe

9b-(2-Aethoxyäthoxy)-1,9b-dihydro-3-isopropyl-7-(methoxymethyl)-3-methyl-2H-imidazo[1',2':1,2]pyrrolo[3,4-b]-pyridin--2,5(3H)-dion,

7-Aethoxy-9b-(2-äthoxyäthoxy)-1,9b-dihydro-3-isopropyl-3--methyl-8-methylthio-2H-imidazo[1',2':1,2]pyrrolo[3,4-b]pyridin-2,5(3H)-dion,

7-Aethoxy-9b-(2-äthoxyäthoxy)-1,9b-dihydro-3-isopropyl-3--methyl-8-phenylthio-2H-imidazo[1',2':1,2]pyrrolo[3,4-b]pyridin-2,5(3H)-dion,

7-Aethoxy-9b-(2-äthoxyäthoxy)-8-(2-chlor-4-trifluormethyl-phenoxy)-1,9b-dihydro-3-isopropyl-3-methyl-2H-imidazo[1',2'-:1,2]pyrrolo[3,4-b]pyridin-2,5(3H)-dion,

9'b-(2-Aethoxyäthoxy)-1',9'b-dihydro-spiro(2,2-dimethylcyclopropan)-1,3'(5'H)-imidazo[1',2':1,2]pyrrolo[3,4-b]pyridin-2'-,5'(3'H)-dion,

7-Aethyl-1,9b-dihydro-9b-hydroxy-3-isopropyl-3-methyl-2H--imidazo[1',2':1,2]pyrrolo[3,4-b]pyridin-2,5(3H)-dion,

7-Aethoxy-1,9b-dihydro-9b-hydroxy-3-isopropyl-3-methyl-2-H-imidazo[1',2':1,2]pyrrolo[3,4-b]pyridin-2,5(3H)-dion und

1',9'b-Dihydro-9'b-hydroxy-spirocyclopentan-1,3'-[2H]-imidazo[1',2':1,2]pyrrolo[3,4-b]pyridin-2,5(3H)-dion ausgewählten Verbindung sowie Formulierungshilfsstoffe enthält.

15. Verfahren zur Herstellung von Verbindungen der allgemeinen Formel

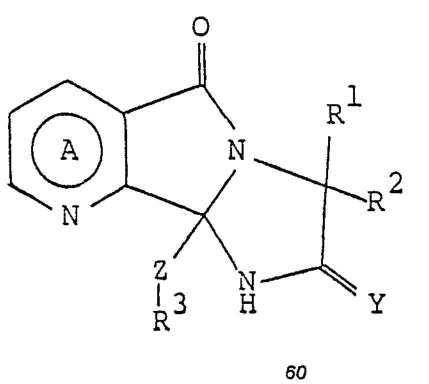

I

worin der

Pyridinring A gegebenenfalls substituiert sein kann, und $R^1$ gegebenenfalls mit Fluor und/oder Chlor mono- oder mehrfach substituiertes $C_{1-4}$-Alkyl,

$R^2$ $C_{1-10}$-Alkyl oder $C_{3-6}$-Cycloalkyl, oder

$R^1$ und $R^2$ zusammen mit dem Kohlenstoffatom, an das sie geknüpft sind, einen gegebenenfalls mit einem oder zwei $C_{1-4}$-Alkylresten substituierten $C_{3-6}$-Cycloalkanring,

$R^3$ Wasserstoff; unsubstituiertes verzweigtes Alkyl mit bis zu 12 Kohlenstoffatomen; $C_{1-12}$-Alkyl, substituiert mit einem oder mehreren Halogenatomen, einer oder mehreren Hydroxylgruppen, einer Cyanogruppe, einer $C_{3-6}$-Cycloalkylgruppe, einer $C_{1-4}$-Alkoxygruppe, einer Pyridylgruppe, einer gegebenenfalls substituierten Phenoxygruppe, einer α- oder β-Naphthyloxygruppe oder einer der Gruppen (c)-(j)

-CO-$R^6$ (c)

worin

$R^6$ Wasserstoff, Hydroxy, $C_{1-4}$-Alkyl, $C_{1-4}$-Alkoxy oder Phenyl bedeutet,

-$SO_{n'}R^7$ (d)

worin

$R^7$ Hydroxy, Methyl, Phenyl oder p-Tolyl und

n' 0, 1 oder 2 bedeuten,

wobei, falls $R^7$ Hydroxy bedeutet, n' 2 bedeutet,

-$OSO_2R^7$ (e)

worin $R^7$ die oben angegebene Bedeutung besitzt,

einer gegebenenfalls veresterten Phosphit-, Phosphat- oder (f)

Phosphonatgruppe

-$NHCONHR^9$ (g)

worin $R^9$ Wasserstoff, $C_{1-4}$-Alkyl oder Phenyl bedeutet,

-$NHCOOR^{10}$ (h)

worin $R^{10}$ $C_{1-4}$-Alkyl bedeutet,

-$OCONHR^9$ (i)

worin $R^9$ die oben angegebene Bedeutung besitzt,

-$OCO(CH_2)_{n'}COR^{11}$ (j)

worin

$R^{11}$ Hydroxy, $C_{1-4}$-Alkoxy, Phenoxy oder Benzyloxy bedeutet und

n' die oben angegebene Bedeutung besitzt,

$C_{3-10}$-Alkinyl; $C_{3-6}$-Cycloalkyl; oder eine Gruppe (a) oder (b)

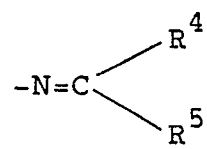

(a)

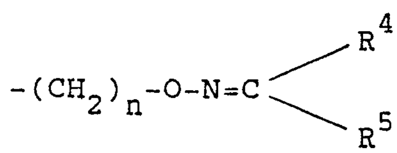

(b)

$R^4$ und $R^5$ unabhängig voneinander $C_{1-4}$-Alkyl,

n 1 oder 2 und

Y und Z unabhängig voneinander Sauerstoff oder Schwefel bedeuten,

mit Ausnahme von 1,9b-Dihydro-9b-hydroxy-3-isopropyl-3-methyl-2-thioxo-2H-imidazo[1',2':1,2]pyrrolo[3,4-b]pyridin-5(3H)-on sowie von 1,11b-Dihydro-11b-hydroxy-3-isopropyl-3-methyl-2H-imidazo[1',2':1,2]pyrrolo[3,4-b]chinolin-2,5(3H)-dion, dadurch gekennzeichnet, dass man eine Verbindung der allgemeinen Formel

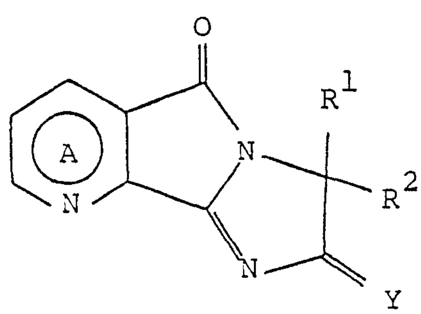

II

worin der Ring A, $R^1$, $R^2$ und Y die oben angegebenen Bedeutungen besitzen,

mit einer Verbindung der allgemeinen Formel

$R^3ZH$ III

worin $R^3$ und Z die oben angegebenen Bedeutungen besitzen,

umsetzt.

16. Verfahren zur Herstellung eines Unkrautbekämpfungsmittels, dadurch gekennzeichnet, dass man mindestens eine der in Anspruch 1 genannten Verbindungen mit Formulierungshilfsstoffen vermischt.

17. Verfahren zur Bekämpfung von Unkräutern, dadurch gekennzeichnet, dass man das gegen Unkräuter zu schützende Gut und/oder die Unkräuter mit einer wirksamen Menge mindestens einer in einem der Ansprüche 1 bis 6, 9, 12 und 13 beschriebenen Verbindung bzw. eines Mittels gemäss einem dieser Ansprüche behandelt.

18. Verfahren zur Bekämpfung von Unkräutern, dadurch gekennzeichnet, dass man das gegen Unkräuter zu schützende Gut und/oder die Unkräuter mit einer wirksamen Menge mindestens einer in einem der Ansprüche 7, 8, 10, 11 und 14 beschriebenen Verbindung bzw. eines Mittels gemäss einem dieser Ansprüche behandelt.

19. Verwendung einer in einem der Ansprüche 1 bis 6, 9, 12 (und 13 beschriebenen Verbindung bzw. eines Mittels gemäss einem dieser Ansprüche zur Bekämpfung von Unkräutern.

20. Verwendung einer in einem der Ansprüche 7, 8, 10, 11 und 14 beschriebenen Verbindung bzw. eines Mittels gemäss einem dieser Ansprüche zur Bekämpfung von Unkräutern.